# EUROPÄISCHE PATENTSCHRIFT

(11) **EP 4 263 857 B1**
(45) Veröffentlichungstag und Bekanntmachung des Hinweises auf die Patenterteilung: **12.02.2025**
(21) Anmeldenummer: 21834753.2
(22) Anmeldetag: 07.12.2021
(51) Int. Cl.: C12Q 1/6806

(54) **ERMITTLUNG DER QUANTITÄT UND QUALITÄT EINER DNA-BIBLIOTHEK**
DETERMINING THE QUANTITY AND QUALITY OF A DNA LIBRARY
DÉTERMINATION DE LA QUANTITÉ ET DE LA QUALITÉ D'UNE BANQUE D'ADN

(30) Priorität: 17.12.2020 DE 102020216120
(43) Veröffentlichungstag der Anmeldung: 25.10.2023
(73) Patentinhaber: Robert Bosch GmbH, 70442 Stuttgart (DE)
(72) Erfinder: HOFFMANN, Anne, 71065 Sindelfingen (DE); GRUMAZ, Christian, 71336 Waiblingen (DE); LEMUTH, Karin, 8704 Herrliberg (CH)
(86) Internationale Anmeldenummer: PCT/EP2021/084559
(87) Internationale Veröffentlichungsnummer: WO 2022/128615

(56) Entgegenhaltungen:
- WO-A1-2018/183621
- KR-B1- 102 185 443
- US-A1- 2016 289 755
- US-A1- 2019 329 254
- CICERA R. LAZZAROTTO ET AL: "Defining CRISPR–Cas9 genome-wide nuclease activities with CIRCLE-seq", NATURE PROTOCOLS, vol. 13, no. 11, 19 October 2018 (2018-10-19), GB, pages 2615 - 2642, XP055556001, ISSN: 1754-2189, DOI: 10.1038/s41596-018-0055-0
- LEGGATE JOHANNA ET AL: "An Internal Amplification Control System Based on Primer-Dimer Formation for PCR Product Detection by DNA Hybridization", JOURNAL OF FOOD PROTECTION, vol. 69, no. 9, 1 September 2006 (2006-09-01), pages 2280 - 2284, XP055903831, Retrieved from the Internet <URL:https://meridian.allenpress.com/jfp/article/69/9/2280/171449/An-Internal-Amplification-Control-System-Based-on>

## Beschreibung

Die vorliegende Erfindung bezieht sich auf eine adapterdimerspezifische Sonde sowie ein Verfahren zur Detektion von Adapterdimeren in einer Desoxyribonukleinsäure (deoxyribonucleic acid, DNA) -Bibliothek, nach dem Oberbegriff der unabhängigen Ansprüche.

### Stand der Technik

Die Publikation von Hardigan et al. CRISPR/Cas9-targeted Removal of Unwanted Sequences From small-RNA Sequencing Libraries. Nucleic Acids Research 2019 betrifft die Entfernung von Adapterdimeren aus Nukleinsäure-Bibliotheken. Die Entfernung erfolgt durch eine spezifische Spaltung der Adapterdimere mit Hilfe des CRISPR/Cas9-Systems. Zur Überprüfung der erfolgreichen Entfernung der Adapterdimere ist eine Quantifizierung der Adapterdimere mittels quantitativer PCR beschrieben. Dabei werden Adapter-überlappende Primer verwendet. Die Publikation von Shore et al. Small RNA Library Preparation Method for Next-Generation Sequencing Using Chemical Modifications to Prevent Adapter Dimer Formation. PLoS One 2016 betrifft Adaptermoleküle zum Erstellen von sequenzierbaren Nukleinsäure-Bibliotheken unter Vermeidung der Bildung von Adapterdimeren.

WO 2014/144979 A1 offenbart eine Reduktion oder Inhibierung der Bildung von Adapterdimeren bei der Präparation von Nukleinsäure-Bibliotheken.

WO 2019/222706 A1 betrifft die Sequenzierung von genomischer DNA. Es werden Verfahren bereitgestellt, die sich auf Polynukleotid-Bibliotheken beziehen.

WO 2009/106308 A2 beschreibt die Verarbeitung von Nukleinsäuren zum Erstellen von sequenzierbaren Nukleinsäure-Bibliotheken.

WO 2015/044412 A1 offenbart Adaptermoleküle zum Erstellen von sequenzierbaren Nukleinsäure-Bibliotheken und das Vermeiden der Bildung von Adapterdimeren.

US 2016/289755 A1 offenbart eine Abschätzung von Adapterdimeren in NGS-Bibliotheken mittels ddPCR.

Zur Sequenzierung von DNA-Proben werden heute nahezu ausschließlich Sequenziermethoden der nächsten Generation (engl. "Next Generation Sequencing, NGS) angewandt. Mit dem Begriff NGS werden eine ganze Reihe von verschiedenen Verfahren zur Sequenzierung bezeichnet, welche sich alle im Wesentlichen dadurch auszeichnen, dass automatisiert eine größere Menge von Untersuchungen parallel und in kurzer Zeit durchgeführt werden können. Mittels NGS ist es beispielsweise möglich, neue krankheitsrelevante Gene und krankheitsverursachende genetische Mutationen, also Veränderungen im Erbgut, zu entdecken. Solche Erkenntnisse helfen, grundlegende biologische Prozesse zu verstehen und Informationen über das Zustandekommen von Erbkrankheiten zu gewinnen.

Eine der NGS-Methoden ist die Amplikonsequenzierung (engl. amplicon sequencing oder targeted sequencing), bei welcher die Fragmentlängen der zu sequenzierenden DNA-Fragmente bereits vor dem Sequenzierlauf aufgrund entsprechender Probenvorbereitung bekannt sind.

Beim NGS erfolgen vor jedem Sequenzierlauf eine genaue Quantifizierung sowie Qualitätsanalyse der DNA-Bibliothek um einen erfolgreichen Sequenzierlauf gewährleisten zu können. Bei der Quantifizierung wird die Konzentration der Fragmente in der DNA-Bibliothek ermittelt. Bei der Qualitätsanalyse erfolgt beispielsweise die Ermittlung der Fragmentlängen der zu sequenzierenden DNA-Fragmente sowie die Ermittlung der Bildung ungewollter Adapterdimere oder anderer Artefakte. Quantifizierung und Qualitätsanalyse erfolgen unabhängig voneinander mittels verschiedener Methoden und Technologien.

### Offenbarung der Erfindung

Erfindungsgemäß werden eine adapterdimerspezifische Sonde sowie ein Verfahren zur Detektion von Adapterdimeren in einer DNA-Bibliothek, mit den kennzeichnenden Merkmalen der unabhängigen Ansprüche bereitgestellt.

Die verfügbaren NGS-Methoden unterscheiden sich zwar in den zu verwendenden Reagenzien und Protokollen, die wesentlichen Verfahrensschritte sind jedoch allen NGS-Methoden gleich. Zunächst muss die zu sequenzierende DNA für die Sequenzierung vorbereitet werden. Hierzu wird die zu sequenzierende DNA-Probe enzymatisch oder mechanisch in kleine Fragmente geschnitten. Die Fragmentlänge der DNA-Fragmente ist von der jeweiligen Methode abhängig. Anschließend werden kurze, künstlich hergestellte als Adaptermoleküle bezeichnete DNA-Stücke an die beiden freien Enden der DNA-Fragmente angehängt, meist durch einen Ligationsschritt. Die Adaptermoleküle sind so aufgebaut, dass sie eine komplementäre Nukleotidsequenz zu verwendeten Primern aufweisen. Primer sind Oligonukleotide mit bekannter Nukleotidsequenz, welche für DNA-replizierende Enzyme wie beispielsweise die DNA-Polymerase als Startpunkt dienen.

Um für eine Sequenzierung eine ausreichende Kopienanzahl an DNA-Fragmenten bereitstellen zu können, werden die mit Adaptermolekülen flankierten DNA-Fragmente beispielsweise mittels Polymerase-Kettenreaktion (engl. polymerase chain reaction, PCR) -basierten Verfahren vervielfältigt. Liegt die DNA fragmentiert, mit Adaptermolekülen versehen und spezifisch angereichert vor, spricht man von einer DNA-Bibliothek (DNA-Library).

Zur Quantifizierung der DNA-Bibliothek eignet sich beispielsweise besonders die quantitative Echtzeit-PCR (engl. real-time quantitative PCR, qPCR) in welcher die amplifizierten DNA-Fragmente während eines PCR-Zyklus in Echtzeit quantifiziert werden. Die Quantifizierung erfolgt beispielsweise durch Fluoreszenzmessungen. Fluoreszenzfarbstoffe lagern sich in die DNA-Fragmente ein, wodurch die Fluoreszenz proportional mit der Menge an amplifizierten DNA-Fragmenten zunimmt.

Bei der Herstellung von DNA-Bibliotheken binden die Adaptermoleküle nicht nur an die freien Enden der zu amplifizierenden DNA-Fragmente, sondern können sich auch miteinander verbinden, sodass Adapterdimere entstehen. Diese sind ungewollt und verfälschen das Ergebnis sowohl bei der Quantifizierung der Fragmente in der DNA-Bibliothek als auch bei einer späteren Sequenzierung. Bei der Quantifizierung erscheint die Anzahl der mit Adaptermolekülen flankierten DNA-Fragmente im Falle des Vorhandenseins von Adapterdimeren höher, als sie wirklich ist, da auch die Adapterdimer-Fragmente mit quantifiziert werden. Um die Quantifizierung der Fragmente in einer DNA-Bibliothek bewerten zu können und die Entstehung von Artefakten wie beispielsweise Adapterdimeren kontrollieren zu können, müssen somit zusätzlich zu der qPCR weitere Methoden zu Überprüfung der Qualität durchgeführt werden.

Gemäß der Erfindung wird eine adapterdimerspezifische Sonde zur Detektion von Adapterdimeren, insbesondere in einer DNA-Bibliothek, offenbart. Die adapterdimerspezifische Sonde umfasst eine Markierung aufgrund derer sie detektierbar ist. Diese Markierung ist insbesondere ein Fluorophor, sodass eine Fluoreszenzstrahlung detektiert wird wenn das Fluorophor mit Licht einer bestimmten Wellenlänge angeregt wird. Alternativ ist die Markierung der adapterdimerspezifischen Sonde eine chemilumineszente Markierung mittels welcher eine Lichtemission detektiert wird als Ergebnis einer chemischen Reaktion (Chemilumineszenz) oder einer enzymatischen Reaktion (Biolumineszenz). Weiterhin alternativ ist die Markierung der adapterdimerspezifischen Sonde eine radioaktive Markierung, bei welcher die radioaktive Strahlung detektiert wird oder eine redoxaktive Komponente, deren Aktivität durch elektrochemische Methoden detektiert wird.

Die adapterdimerspezifische Sonde ist beispielsweise eine so genannte Taqman-Sonde, welche einen Signalunterdrücker (engl. Quencher) umfasst. Im Fall eines Fluorophors als Markierung beispielsweise löscht der Signalunterdrücker das Fluoreszenzsignal des Fluorophors, indem das Fluorophor zumindest einen Teil seiner Energie an den Signalunterdrücker abgibt, wenn es durch eine Lichtquelle angeregt wird.

Die Unterdrückung erfolgt nur, wenn der Signalunterdrücker und das Fluorophor sich in unmittelbarer räumlicher Nähe zueinander befinden. Bei einer DNA-Amplifikation insbesondere des komplementären DNA-Strangs, beispielsweise durch eine Polymerase, wird die kovalent an den DNA-Strang gebundene Sonde abgebaut. Dadurch entfernen sich der Signalunterdrücker und das Fluorophor voneinander, und eine steigende Fluoreszenz des Fluorophors kann gemessen werden.

Die adapterdimerspezifische Sonde ist ausgebildet, spezifisch an eine erste Adapternukleotidsequenz eines ersten Adaptermoleküls und an eine zweite Adapternukleotidsequenz eines zweiten Adaptermoleküls zu binden.

Vorteilhaft bei der erfindungsgemäßen adapterdimerspezifischen Sonde ist, dass Adapterdimere direkt und spezifisch durch die adapterdimerspezifische Sonde gebunden werden und somit schnell und einfach detektiert werden können. Es sind somit keine weiteren zeit- und/oder kostenintensive Verfahren zum Nachweis von Adapterdimeren notwendig.

Desweiteren wird ein Verfahren zur Detektion von Adapterdimeren in einer DNA-Bibliothek während einer qPCR offenbart, wobei die DNA-Bibliothek DNA-Fragmente, welche an beiden freien Enden von Adaptermolekülen flankiert sind, und Adapterdimere umfasst.

Das Verfahren weist die nachfolgenden Schritte auf:
a) Zugabe einer adapterdimerspezifischen Sonde zu einem qPCR-Ansatz umfassend die DNA-Bibliothek
b) Bestimmung der Anzahl an enthaltenen Adapterdimeren in der DNA-Bibliothek mittels des von der adapterdimerspezifischen Sonde ausgegebenen Signals
c) Berechnung der tatsächlichen Anzahl an DNA-Fragmenten in der DNA-Bibliothek unter Berücksichtigung der ermittelten Anzahl an Adapterdimeren.

Im Generellen ist die qPCR eine sehr exakte Methode zur Bestimmung der Konzentration an DNA-Fragmenten in der DNA-Bibliothek, da ausschließlich spezifisch adaptergebundene Fragmente amplifiziert und somit quantifiziert werden. Haben sich jedoch auch Adapterdimere gebildet, so binden die Primer bei der qPCR ebenfalls an diese, sodass die Adapterdimere ebenfalls mitquantifiziert werden. In diesem Fall fällt die gemessene DNA-Konzentration höher aus als sie tatsächlich ist.

Somit ist es herkömmlich für eine genaue Quantifizierung erforderlich, die Fragmentlängen der in der DNA-Bibliothek enthaltenen Fragmente über eine weitere Methode, beispielsweise eine Gelelektrophorese oder ggf. Kapillargelelektrophorese, zu bestimmen um daraus die tatsächliche Anzahl an DNA-Fragmenten in der DNA-Bibliothek berechnen zu können. Hierbei wird die Anzahl an Adapterdimeren von der Anzahl an DNA-Fragmenten abgezogen.

Vorteilhaft bei dem erfindungsgemäßen Verfahren ist, dass die Anzahl an Adapterdimeren in der DNA-Bibliothek in Echtzeit direkt während der qPCR mittels der adapterdimerspezifischen Sonde bestimmt wird. Somit sind keine zeitaufwändigen und arbeitsintensiven Methoden, wie beispielsweise eine Bestimmung der Fragmentlängen der DNA-Fragmente und der Adapterdimere in der DNA-Bibliothek notwendig, insbesondere wenn die Fragmentlängen der DNA-Fragmente bekannt sind, wie dies beispielsweise der Fall ist bei einer DNA-Bibliothek für eine Amplikonsequenzierung. Bei bekannten Fragmentlängen der DNA-Fragmente kann die Anzahl an DNA-Fragmenten aus der korrigierten Quantifizierung errechnet werden, ohne dass sich der qPCR weitere Analysemethoden und -verfahren anschließen. Vorteilhaft hierbei ist, dass mittels der erfindungsgemäßen Sonde und des erfindungsgemäßen Verfahrens somit Arbeitsschritte, sowie zeitliche, finanzielle und materielle Ressourcen eingespart werden.

Die ermittelte Anzahl an Adapterdimeren kann darüber hinaus als Qualitätsmerkmal entscheidend dafür sein, ob die DNA-Bibliothek für eine anschließende Sequenzierung genutzt werden kann.

Weitere vorteilhafte Ausführungsformen ergeben sich aus den Unteransprüchen.

In einer besonders vorteilhaften Ausführungsform des Verfahren wird dem qPCR-Ansatz
in Schritt a) desweiteren zumindest eine adapterspezifische Sonde für ein erstes Adaptermolekül zugegeben und
in Schritt b) zusätzlich die Anzahl der enthaltenen ersten Adaptermoleküle mittels des von der adapterspezifischen Sonde ausgegebenen Signals bestimmt und in Schritt c) erfolgt die Berechnung der tatsächlichen Anzahl an DNA-Fragmenten in der DNA-Bibliothek unter Berücksichtigung der ermittelten Anzahl an Adapterdimeren und der ermittelten Anzahl an ersten Adaptermolekülen.

Vorteilhaft hierbei ist, dass sowohl die Anzahl an Adapterdimeren in der DNA-Bibliothek in Echtzeit direkt während der qPCR mittels der adapterdimerspezifischen Sonde nachgewiesen wird, sowie auch die Anzahl an ersten Adaptermolekülen mittels der adapterspezifischen Sonde. Die adapterspezifische Sonde bindet sowohl an erste Adaptermoleküle, welche DNA-Fragmente flankieren, sowie an erste Adaptermoleküle von Adapterdimeren. Mittels der adapterspezifischen Sonde wird somit die Gesamtanzahl an ersten Adaptermolekülen detektiert. Um die tatsächliche Anzahl an DNA-Fragmenten in der DNA-Bibliothek zu berechnen, wird die Anzahl an detektierten Adapterdimeren von der Anzahl an detektierten ersten Adaptermolekülen abgezogen. Es sind somit keine zeitaufwändigen und arbeitsintensiven Methoden zur Bestimmung der tatsächlichen Anzahl an DNA-Fragmenten, wie beispielsweise eine Bestimmung der Fragmentlängen der DNA-Fragmente und der Adapterdimere in der DNA-Bibliothek, notwendig.

Während der qPCR erfolgen also parallel eine Quantifizierung der in der DNA-Bibliothek enthaltenen Fragmente durch das Signal der adapterspezifischen Sonde und eine Bestimmung der Qualität der DNA-Bibliothek, durch das Signal der adapterdimerspezifischen Sonde. Hierdurch werden Arbeitsschritte, sowie zeitliche, finanzielle und materielle Ressourcen eingespart.

Die ermittelte Anzahl an Adapterdimeren kann darüber hinaus als Qualitätsmerkmal entscheidend dafür sein, ob die DNA-Bibliothek für eine anschließende Sequenzierung genutzt werden kann.

In einer weiteren vorteilhaften Ausführungsform des Verfahren wird aufgrund der Berechnung der tatsächlichen Anzahl an DNA-Fragmenten in der DNA-Bibliothek die Menge an einzusetzender DNA für eine anschließende Sequenzierung, insbesondere eine NGS-Sequenzierung, bestimmt.

Die Bestimmung der genauen DNA-Fragmentanzahl in der DNA-Bibliothek für eine DNA-Sequenzierung ist von höchster Bedeutung. Bei einer zu geringer DNA-Beladung auf den Sequenzierer wird nicht das volle Potential ausgeschöpft, was somit unwirtschaftlich ist. Eine zu starke Beladung hat zur Konsequenz, dass die Signale zur Sequenzierung nicht mehr korrekt ausgelesen werden können. Vorteilhaft ist, dass mittels der berechneten tatsächlichen Anzahl an DNA-Fragmenten in der DNA-Bibliothek die optimale Menge an einzusetzender DNA berechnet werden kann und somit eine optimale Beladung des Sequenzierers erfolgt.

Desweiteren ist die ermittelte Anzahl an Adapterdimeren entscheidend dafür ob die DNA-Bibliothek für eine Sequenzierung genutzt werden kann. Bei einem zu hohen Anteil an Adapterdimeren sollte sie nicht verwendet werden.

Es ist von Vorteil, wenn es sich bei dem Verfahren um eine Amplikonsequenzierung handelt, bei der die Länge der DNA-Fragmente bekannt ist.

Vorteilhaft hierbei ist, dass die Länge der DNA-Fragmente durch die spezifische Probenvorbereitung bekannt ist. Je nach qPCR-Ansatz wird dieser Parameter benötigt, um über eine Berechnung der Quantität und Qualität der DNA-Bibliothek die optimalen DNA-Menge für die Amplikonsequenzierung einsetzen zu können. Es entfallen also ggf. durch die Wahl einer Amplikonsequenzierung weitere Verfahren, insbesondere zur Fragmentlängen-Bestimmung der DNA-Fragmente, wodurch Zeit und Kosten sowie Arbeitsschritte eingespart werden.

Gegenstand der Offenbarung, ohne Teil der Erfindung zu sein, ist ferner eine Kartusche, insbesondere eine mikrofluidische Kartusche, wie beispielsweise in DE102016222072A1 oder DE102016222075A1 beschrieben, wobei die Kartusche ausgebildet ist, das erfindungsgemäße Verfahren durchzuführen.

Die beschriebenen Verfahrensschritte zur in Echtzeit Detektion von Adapterdimeren einer DNA-Bibliothek während einer qPCR oder zur in Echtzeit Detektion von Adapterdimeren und von ersten Adaptermolekülen einer DNA-Bibliothek während einer qPCR erfolgen dann beispielsweise alle innerhalb der Kartusche. Ebenfalls innerhalb der Kartusche erfolgt beispielsweise eine anschließende Sequenzierung. Auch für die qPCR vorbereitende Schritte erfolgen beispielsweise innerhalb der Kartusche.

Besonders in einem mikrofluidischen System ist eine kombinierte Quantitäts- und Qualitätskontrolle von Vorteil, da keine weiteren Methoden implementiert werden müssen.

Alternativ erfolgen alle beschriebenen Schritte händisch.

### Kurze Beschreibung der Zeichnung

Ausführungsformen der vorliegenden Erfindung sind in der Zeichnung dargestellt und in der nachfolgenden Figurenbeschreibung näher erläutert. Es zeigt:
Fig. 1: die schematische Darstellung einer erfindungsgemäßen adapterdimerspezifischen Sonde zur Detektion von Adapterdimeren,
Fig. 2: die schematische Darstellung eines erfindungsgemäßen Verfahrens zur Detektion von Adapterdimeren in einer DNA-Bibliothek während einer qPCR in einer ersten Ausführungsform,
Fig. 3: die schematische Darstellung des erfindungsgemäßen Verfahrens zur Detektion von Adapterdimeren in einer DNA-Bibliothek während einer qPCR in einer zweiten Ausführungsform, und
Fig. 4: die schematische Darstellung einer Kartusche, ausgebildet zur Durchführung des Verfahrens gemäß Figur 3.

### Ausführungsformen der Erfindung

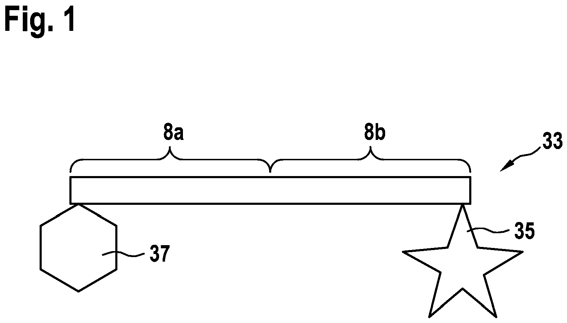

In Figur 1 ist eine erfindungsgemäße adapterdimerspezifische Sonde 33 zur Detektion von Adapterdimeren 3, insbesondere in einer DNA-Bibliothek, dargestellt.

Die adapterdimerspezifische Sonde 33 umfasst eine erste DNA-Nukleotidsequenz 8a, welche komplementär ist zu einem Teil der Adapternukleotidsequenz eines ersten Adaptermoleküls 2a und eine zweite DNA-Nukleotidsequenz 8b, welche komplementär ist zu einem Teil der Adapternukleotidsequenz eines zweiten Adaptermoleküls 2b. Mittels der ersten und zweiten DNA-Nukleotidsequenzen 8a, 8b bindet die adapterdimerspezifische Sonde 33 somit spezifisch an Adapterdimere 3.

Die adapterdimerspezifische Sonde 33 umfasst ein Fluorophor 35 als Markierung. Wird das Fluorophor 35 mit Licht einer bestimmten Wellenlänge angeregt, so emittiert es eine detektierbare Fluoreszenzstrahlung. In einer alternativen, nicht dargestellten Ausführungsform ist die Markierung der adapterdimerspezifischen Sonde 33 eine chemilumineszente Markierung, eine radioaktive Markierung oder eine redoxaktive Komponente.

Die adapterdimerspezifische Sonde 33 ist beispielsweise eine Taqman-Sonde, welche einen Signalunterdrücker 37 umfasst. Der Signalunterdrücker 37 löscht das Fluoreszenzsignal des Fluorophors 35, indem das Fluorophor 35 zumindest einen Teil seiner Energie an den Signalunterdrücker 37 abgibt, wenn es durch die Lichtquelle angeregt wird.

Die Unterdrückung erfolgt nur, wenn der Signalunterdrücker 37 und das Fluorophor 35 sich in räumlicher Nähe zueinander befinden. Bei einer DNA-Amplifikation, beispielsweise durch eine DNA-Polymerase, wird die kovalent an den DNA-Strang gebundene adapterdimerspezifische Sonde 33 abgebaut. Dadurch entfernen sich der Signalunterdrücker 37 und das Fluorophor 35 voneinander, und eine steigende Fluoreszenz des Fluorophors 35 kann gemessen werden.

In einer alternativen Ausführungsform ist die adapterdimerspezifische Sonde 33 eine andere Art von Sonde.

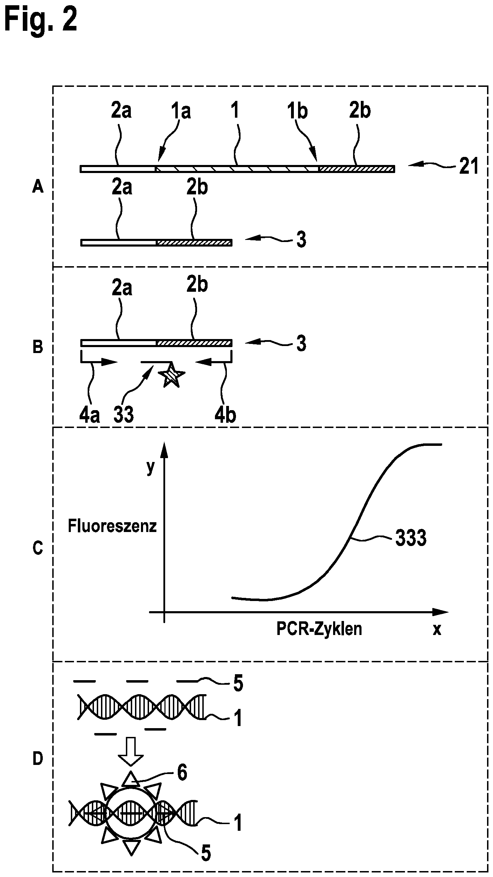

In Figur 2 ist ein erfindungsgemäßes Verfahren zur Detektion von Adapterdimeren 3 in einer DNA-Bibliothek während einer qPCR in einer ersten Ausführungsform dargestellt.

Zunächst, und nicht in Figur 2 dargestellt, wird eine DNA-Probe derart vorbereitet, dass DNA-Fragmente 1 vorliegen. Hierzu wird die DNA-Probe enzymatisch oder mechanisch in DNA-Fragmente 1 zerlegt oder alternativ beispielsweise mittels einer Amplikon-PCR (engl. targeted PCR) bestimmte DNA-Fragmente 1 angereichert. Die Fragmentlängen der DNA-Fragmente 1 sind von der jeweiligen Methode abhängig.

Anschließend, und in Schritt A dargestellt, werden erste Adaptermoleküle 2a und zweite Adaptermoleküle 2b zu den DNA-Fragmenten 1 gegeben.

Die Adaptermoleküle 2a, 2b sind beispielsweise pseudodoppelsträngige Adaptermoleküle 2a, 2b und werden beispielsweise durch eine Ligation asymmetrisch an die beiden freien Enden 1a, 1b der DNA-Fragmente 1 gebracht. Die ersten Adaptermoleküle 2a binden somit spezifisch an das das erste freie Ende 1a der DNA-Fragmente 1 und die zweiten Adaptermoleküle 2b binden somit spezifisch an das zweite freie Ende 1b der DNA-Fragmente 1. Auf diese Weise entstehen von Adaptermolekülen 2a, 2b flankierte DNA-Fragmente 21. Das erste Adaptermolekül 2a weist eine komplementäre Nukleotidsequenz zu einem ersten Primer auf. Das zweite Adaptermolekül 2b weist eine komplementäre Nukleotidsequenz zu einem zweiten Primer auf.

Die Adaptermoleküle 2a, 2b binden nicht nur an die freien Enden der DNA-Fragmente 1, sondern können sich, wenn auch weniger effizient, auch miteinander verbinden, sodass Adapterdimere 3 entstehen. Diese sind ungewollt und verfälschen unter anderem das Ergebnis bei einer späteren Quantifizierung der DNA-Fragmente 1.

In einem weiteren, nicht in der Figur dargestellten Schritt, werden die flankierten DNA-Fragmente 21 und die Adapterdimere 3, beispielsweise mittels einem PCR-Verfahren vervielfältigt.

Hiernach liegt eine DNA-Bibliothek vor, welche DNA-Fragmente 1, die an beiden freien Enden von Adaptermolekülen 2a, 2b flankiert sind, und Adapterdimere 3 umfasst.

Um die Menge an DNA-Fragmenten 1 und Adapterdimeren 3, beispielsweise für Analysen oder für eine Sequenzierung der DNA-Fragmente 1, bestimmen zu können, wird eine qPCR durchgeführt. Vorbereitend hierfür und in Schritt B dargestellt werden dem Ansatz ein erster Primer 4a und ein zweiter Primer 4b zugegeben, welche für DNA-replizierende Enzyme, wie beispielsweise die DNA-Polymerase, als Startpunkt dienen. Der erste Primer 4a bindet spezifisch an die komplementäre Nukleotidsequenz des ersten Adaptermoleküls 2a und der zweite Primer 4b bindet spezifisch an die komplementäre Nukleotidsequenz des zweiten Adaptermoleküls 2b. Hierbei ist die zu den Primern 4a, 4b komplementäre Nukleotidsequenz der Adaptermoleküle 2a, 2b beispielsweise nur ein Teilbereich der Nukleotidsequenz der Adaptermoleküle 2a, 2b an den jeweils ungebundenen Enden der Adaptermoleküle 2a, 2b. Desweiteren wird dem qPCR-Ansatz eine adapterdimerspezifische Sonde 33, wie sie beispielsweise zu Figur 1 beschrieben ist, hinzugegeben.

Die adapterdimerspezifische Sonde 33 bindet spezifisch an eine erste Adapternukleotidsequenz eines ersten Adaptermoleküls 2a und an eine zweite Adapternukleotidsequenz eines zweiten Adaptermoleküls 2b. Somit ist die adapterdimerspezifische Sonde 33 ausgebildet, spezifisch an Adapterdimere 3 zu binden.

Bei der sich anschließenden qPCR werden die flankierten DNA-Fragmente 21 sowie die Adapterdimere 3 vervielfältigt. Während der DNA-Amplifikation wird nach jedem Zyklus die von der adapterdimerspezifischen Sonde 33 emittierte Fluoreszenz gemessen und eine wie in Schritt C dargestellte adapterdimerspezifische Fluoreszenzkurve 333 aufgenommen. Auf der x-Achse sind die PCR-Zyklen dargestellt und auf der y-Achse die gemessene Fluoreszenz. Mittels einer Standardkurve wird den Fluoreszenzsignalen eine Molekülanzahl zugewiesen. Dadurch lässt sich die Anzahl an Adapterdimeren 3 berechnen.

Zudem werden die amplifizierten DNA-Fragmente 1 nach jedem PCR-Zyklus in Echtzeit auch quantifiziert. In Schritt D oben ist ein DNA-Fragment 1 abgebildet, sowie eine DNA-interkalierende Markierung 5. Die DNA-interkalierende Markierung 5, beispielsweise ein Fluoreszenzfarbstoff, lagert sich wie in Schritt 4 unten dargestellt in die DNA-Fragmente 1 ein, wodurch das Signal 6 der Markierung 5, insbesondere das Fluoreszenzsignal, proportional mit der Menge an amplifizierten DNA-Fragmenten 1 zunimmt. Zudem und nicht in den Figuren dargestellt lagert sich die DNA-interkalierende Markierung 5 auch in die Adapterdimere 3 ein. Es wird hier also die Gesamtkonzentration aus DNA-Fragmenten 1 und Adapterdimeren 3 detektiert. Hieraus kann eine korrigierte Quantifizierung berechnet werden, indem die Konzentration an Adapterdimeren 3 von der Konzentration an detektierter Gesamt-DNA abgezogen wird. Sind die Fragmentlängen der DNA-Fragmente 1 bekannt, wie beispielsweise bei einer Probenvorbereitung für eine Amplikonsequenzierung, so kann die tatsächliche Anzahl an DNA-Fragmenten 1 in der DNA-Bibliothek berechnet werden.

Die ermittelte Anzahl an Adapterdimeren 3 kann darüber hinaus als Qualitätsmerkmal entscheidend dafür sein, ob die DNA-Bibliothek für eine anschließende Sequenzierung genutzt werden kann.

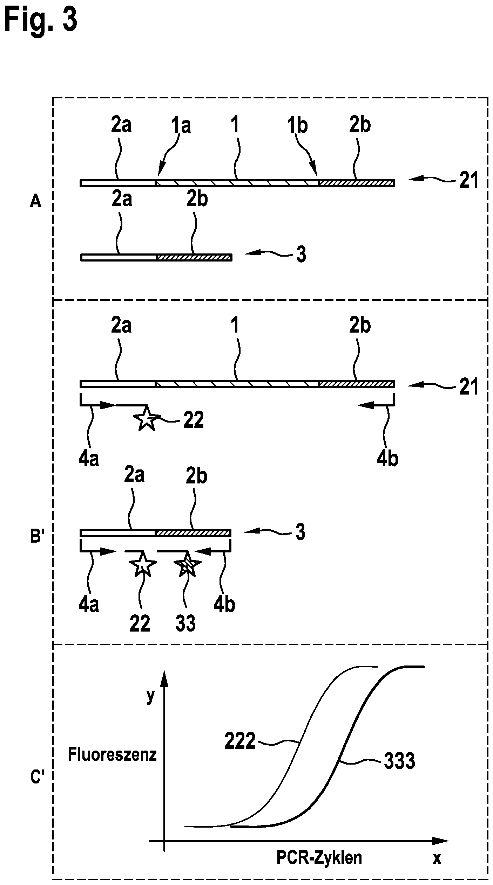

In Figur 3 ist ein erfindungsgemäßes Verfahren zur Detektion von Adapterdimeren 3 in einer DNA-Bibliothek während einer qPCR in einer zweiten Ausführungsform dargestellt.

Im Unterschied zu dem zu Figur 2 beschriebenen Verfahren in der ersten Ausführungsform wird dem qPCR-Ansatz neben der adapterdimerspezifischen Sonde 33 in Schritt B' auch eine adapterspezifische Sonde 22 hinzugegeben. Die adapterspezifische Sonde 22 bindet spezifisch an eine erste Adapternukleotidsequenz eines ersten Adaptermoleküls 2a. Somit bindet die adapterspezifische Sonde 22 sowohl an ein erstes Adaptermolekül 2a welches ein DNA-Fragment 1 flankiert, sowie an ein erstes Adaptermolekül 2a eines Adapterdimers 3. Mittels der adapterspezifischen Sonde 22 ist somit die Gesamtanzahl an ersten Adaptermolekülen 2a detektierbar, welche repräsentativ für die Gesamtanzahl an flankierten DNA-Fragmenten 21 steht.

Bei der sich anschließenden qPCR werden die flankierten DNA-Fragmente 21 sowie die Adapterdimere 3 vervielfältigt. Während der DNA-Amplifikation wird nach jedem Zyklus die von der adapterdimerspezifischen Sonde 33 emittierte Fluoreszenz detektiert und eine wie in Schritt C' dargestellte adapterdimerspezifische Fluoreszenzkurve 333 aufgenommen.

Zusätzlich wird nach jedem Amplifikations-Zyklus die von der adapterspezifischen Sonde 22 emittierte Fluoreszenz detektiert und eine wie in Schritt C' dargestellte adapterspezifische Fluoreszenzkurve 222 aufgenommen. Auf der x-Achse sind jeweils die PCR-Zyklen dargestellt und auf der y-Achse die gemessene Fluoreszenz.

Mittels einer jeweiligen Standardkurve wird den adapterdimer-bezogenen Fluoreszenzsignalen und den adapter-bezogenen Fluoreszenzsignalen jeweils eine Molekülanzahl zugewiesen. Dies erfolgt parallel zur Aufnahme der Fluoreszenzkurven 222, 333. Um hieraus die tatsächliche Anzahl an DNA-Fragmenten 1 in der DNA-Bibliothek zu berechnen, wird die ermittelte Anzahl an Adapterdimeren 3 von der Anzahl an ermittelten ersten Adaptermolekülen 2a abgezogen.

Alternativ, und nicht in Figur 3 dargestellt bindet die adapterspezifische Sonde 22 spezifisch an eine Adapternukleotidsequenz eines zweiten Adaptermoleküls 2b. Die tatsächliche Anzahl an DNA-Fragmenten 1 in der DNA-Bibliothek ist beispielsweise relevant zur Bestimmung der Menge an einzusetzenden DNA-Molekülen für eine anschließende Sequenzierung, insbesondere für eine NGS-Sequenzierung.

Die ermittelte Anzahl an Adapterdimeren 3 kann darüber hinaus als Qualitätsmerkmal entscheidend dafür sein, ob die DNA-Bibliothek für eine anschließende Sequenzierung genutzt werden kann.

Je nach Anzahl an beispielsweise gleichzeitig in den zu den Figuren 2 und 3 beschriebenen Verfahren eingesetzten unterschiedlichen DNA-Fragmenten 1 und somit auch unterschiedlichen eingesetzten Adaptermolekülen 2a, 2b können sich unterschiedliche Artefakte, insbesondere Adapterdimere 3 bilden. Selbstverständlich können dann eine Mehrzahl an unterschiedlichen chimärsequenzspezifischen Sonden zur Detektion von Artefakten, wie beispielsweise der adapterdimerspezifische Sonde 33 zur Detektion von Adapterdimeren 3, in den Verfahren eingesetzt werden. Zur Detektion der Adaptermoleküle 2a,2b können dann beispielsweise mehrere jeweilige adapterspezifische Sonden 22 eingesetzt werden.

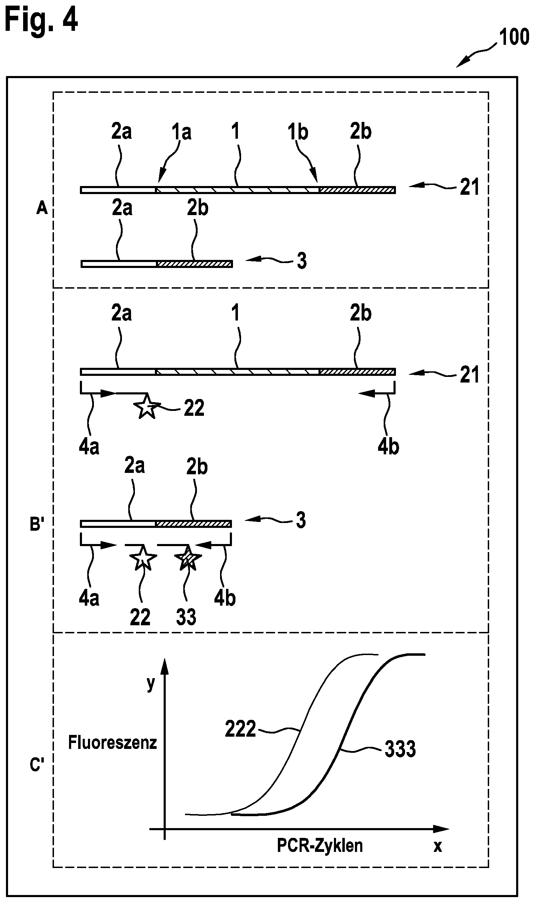

Figur 4 zeigt eine, insbesondere mikrofluidische, Kartusche 100. Die Kartusche 100 ist ausgebildet, ein Verfahren nach der zweiten Ausführungsform auszuführen wie vorstehend zu Figur 3 beschrieben.

Die zu Figur 3 beschriebenen Schritte erfolgen dann beispielsweise alle innerhalb der Kartusche 100. Ebenfalls innerhalb der Kartusche 100 erfolgt beispielsweise eine anschließende Sequenzierung.

Alternativ und nicht in Figur 4 dargestellt, ist die Kartusche 100 ausgebildet, ein Verfahren nach der ersten Ausführungsform wie zu Figur 2 beschrieben, insbesondere mit anschließender Sequenzierung, auszuführen.

In einer alternativen Ausführungsform erfolgen alle beschriebenen Schritte händisch.

## Patentansprüche

1. Adapterdimerspezifische Sonde (33) zur Detektion von Adapterdimeren (3), insbesondere in einer DNA-Bibliothek, wobei die Sonde (33) eine Markierung, insbesondere ein Fluorophor (35), aufweist und ausgebildet ist, spezifisch an eine erste Adapternukleotidsequenz eines ersten Adaptermoleküls (2a) und an eine zweite Adapternukleotidsequenz eines zweiten Adaptermoleküls (2b) zu binden.

2. Verfahren zur Detektion von Adapterdimeren (3) in einer DNA-Bibliothek während einer qPCR, wobei die DNA-Bibliothek umfasst:
- DNA-Fragmente (1), welche an beiden freien Enden von Adaptermolekülen (2a, 2b) flankiert sind, sowie
- Adapterdimere (3)
und wobei das Verfahren nachfolgende Schritte aufweist:
a) Zugabe einer adapterdimerspezifischen Sonde (33) gemäß Anspruch 1 zu einem qPCR-Ansatz umfassend die DNA-Bibliothek
b) Bestimmung der Anzahl an enthaltenen Adapterdimeren (3) in der DNA-Bibliothek mittels des von der adapterdimerspezifischen Sonde (33) ausgegebenen Signals
c) Berechnung der tatsächlichen Anzahl an DNA-Fragmenten (1) in der DNA-Bibliothek unter Berücksichtigung der ermittelten Anzahl an Adapterdimeren (3).

3. Verfahren nach Anspruch 2, **dadurch gekennzeichnet, dass** dem qPCR-Ansatz
in Schritt a) desweiteren zumindest eine adapterspezifische Sonde (22) für ein erstes Adaptermolekül (2a) zugegeben wird und
in Schritt b) zusätzlich die Anzahl der enthaltenen ersten Adaptermoleküle (2a) mittels des von der adapterspezifischen Sonde (22) ausgegebenen Signals bestimmt wird und
in Schritt c) die Berechnung der tatsächlichen Anzahl an DNA-Fragmenten (1) in der DNA-Bibliothek unter Berücksichtigung der ermittelten Anzahl an Adapterdimeren (3) und der ermittelten Anzahl an ersten Adaptermolekülen (2a) erfolgt.

4. Verfahren nach einem der Ansprüche 2 oder 3, **dadurch gekennzeichnet, dass** aufgrund der Berechnung der tatsächlichen Anzahl an DNA-Fragmenten (1) in der DNA-Bibliothek die Menge an einzusetzender DNA für eine anschließende Sequenzierung, insbesondere eine NGS-Sequenzierung, bestimmt wird.

5. Verfahren nach Anspruch 4, **dadurch gekennzeichnet, dass** es sich um eine Amplikonsequenzierung handelt, bei der die Länge der DNA-Fragmente (1) bekannt ist.

## Claims

1. Adapter dimer-specific probe (33) for detection of adapter dimers (3), in particular in a DNA library, wherein the probe (33) comprises a label, in particular a fluorophore (35), and is designed to bind specifically to a first adapter nucleotide sequence of a first adapter molecule (2a) and to a second adapter nucleotide sequence of a second adapter molecule (2b).

2. Method for detecting adapter dimers (3) in a DNA library during a qPCR, wherein the DNA library comprises:
- DNA fragments (1) flanked by adapter molecules (2a, 2b) at both free ends, and
- adapter dimers (3)
and wherein the method comprises the following steps:
a) adding an adapter dimer-specific probe (33) according to Claim 1 to a qPCR reaction mixture comprising the DNA library
b) determining the number of adapter dimers (3) present in the DNA library by means of the signal produced by the adapter dimer-specific probe (33)
c) calculating the actual number of DNA fragments (1) in the DNA library, taking into account the determined number of adapter dimers (3).

3. Method according to Claim 2, **characterized in that** the qPCR reaction mixture
in step a) further receives the addition of at least one adapter-specific probe (22) for a first adapter molecule (2a) and
in step b) additionally the number of first adapter molecules (2a) present is determined by means of the signal produced by the adapter-specific probe (22) and
in step c) the calculation of the actual number of DNA fragments (1) in the DNA library is carried out taking into account the determined number of adapter dimers (3) and the determined number of first adapter molecules (2a).

4. Method according to either of Claims 2 and 3, **characterized in that** the amount of DNA to be used for subsequent sequencing, in particular NGS sequencing, is determined on the basis of the calculation of the actual number of DNA fragments (1) in the DNA library.

5. Method according to Claim 4, **characterized in that** the sequencing is amplicon sequencing in which the length of the DNA fragments (1) is known.

## Revendications

1. Sonde (33) spécifique à des dimères adaptateurs pour la détection de dimères adaptateurs (3), en particulier dans une bibliothèque DNA, la sonde (33) présentant un marqueur, en particulier un fluorophore (35), et étant configurée pour se lier spécifiquement à une première séquence de nucléotides adaptateurs d'une première molécule adaptatrice (2a) et à une deuxième séquence de nucléotides adaptateurs d'une deuxième molécule adaptatrice (2b).

2. Procédé de détection de dimères adaptateurs (3) dans une bibliothèque DNA pendant une PCR quantitative, la bibliothèque DNA comprenant :
- des fragments DNA (1) qui sont encadrés aux deux extrémités libres par des molécules adaptatrices (2a, 2b), ainsi que
- des dimères adaptateurs (3)
et le procédé comprenant les étapes suivantes :
a) ajout d'une sonde (33) spécifique à des dimères adaptateurs selon la revendication 1 à une préparation de PCR quantitative comprenant la bibliothèque DNA
b) détermination du nombre de dimères adaptateurs (3) contenus dans la bibliothèque DNA au moyen du signal délivré par la sonde (33) spécifique à des dimères adaptateurs
c) calcul du nombre réel de fragments DNA (1) dans la bibliothèque DNA en tenant compte du nombre déterminé de dimères adaptateurs (3).

3. Procédé selon la revendication 2, **caractérisé en ce qu'**à la préparation de PCR quantitative
à l'étape a), est ajoutée en plus au moins une sonde (22) spécifique à l'adaptateur pour une première molécule adaptatrice (2a) et
à l'étape b), le nombre des premières molécules adaptatrices (2a) contenues est en plus déterminé au moyen du signal émis par la sonde (22) spécifique à l'adaptateur et
à l'étape c), le calcul du nombre réel de fragments DNA (1) dans la bibliothèque DNA est effectué en tenant compte du nombre déterminé de dimères adaptateurs (3) et du nombre déterminé de premières molécules adaptatrices (2a).

4. Procédé selon l'une des revendications 2 ou 3, **caractérisé en ce que** la quantité DNA à utiliser pour un séquençage ultérieur, en particulier un séquençage NGS, est déterminée sur la base du calcul du nombre réel de fragments DNA (1) dans la bibliothèque DNA.

5. Procédé selon la revendication 4, **caractérisé en ce qu'**il s'agit d'un séquençage d'amplicons avec lequel la longueur des fragments DNA (1) est connue.
